# EUROPEAN PATENT APPLICATION

(11) **EP 3 854 814 A1**
(43) Date of publication of application: **28.07.2021**
(21) Application number: 19801095.1
(22) Date of filing: 20.09.2019
(51) Int. Cl.: C07K 16/02

(54) **PURIFICATION OF IMMUNOGLOBULIN Y AND USES THEREOF**

(30) Priority: 20.09.2018 PT 2018115031
(71) Applicant: Universidade de Aveiro, 3810-193 Aveiro (PT)
(72) Inventor: FREIRE MARTINS, Mara Guadalupe, 3810-696 Aveiro (PT); RODRIGUES ALMEIDA ROCHA, Ana Mafalda, 2500-118 Caldas Da Rainha (PT); SANTOS RESENDE, Judite Sofia, 2840-572 Aldeia De Paio Pires (PT); MORA TAVARES, Ana Paula, 3850 581 Albergaria A Velha (PT); DIMAS NEVES DOMINGUES, Pedro Miguel, 3810-416 Aveiro (PT)
(74) Representative: Patentree
(86) International application number: PCT/IB2019/057994
(87) International publication number: WO 2020/058937

(57) **Abstract**

The present disclosure relates to a process for purifying immunoglobulin Y (IgY) from egg yolk, in particular from chicken egg yolk, with high purity, high stability and high activity, and at a competitive price. The present process makes it possible to obtain approximately 95% pure IgY in a simple, economical and industrially applicable manner.

## Description

### Technical Domain

The present disclosure relates to a process for purifying immunoglobulin Y (IgY) from egg yolk, in particular chicken egg yolk with high purity, high stability and high activity, and at a competitive price. The present process makes it possible to obtain approximately 95% pure IgY in a simple, economical and industrially applicable manner.

### Background Art

The increase of antibiotic resistant microorganisms, the diminishing efficacy thereof and the increasing number of immunocompromised individuals who do not respond to traditional treatments are factors currently having a high impact on health and economy.

For these reasons, there is a high demand need for alternative therapies, such as for example antibody-based biopharmaceuticals. The ability of antibodies to bind to an antigen with high affinity and specificity has led to their use in numerous scientific and medicinal applications (Kovacs-Nolan, et al., Avian Poult. Biol. Ver., 2004, 15, 25-46).

However, the cost of producing high purity antibodies, namely immunoglobulin Y (IgY, an antibody present in poultry egg yolk), remains significantly higher than that of other therapies due to the lack of an effective and profitable purification method.

IgY is a polyclonal antibody functionally equivalent to polyclonal IgG from mammalian serum; however, the amount of specific IgY that can be obtained is higher, with no limitations on egg collection. Each year an immunized chicken is able to produce more than 40 g of IgY through its eggs, which is equivalent to the amount of IgG produced by 40 rabbits. However, the egg is a complex matrix containing about 50% of dry matter. Of this, 2/3 are lipids and 1/3 are proteins. For these reasons, traditional IgY purification techniques hardly allow this antibody to be obtained with high stability and activity, high purity and in large quantities.

IgY purification processes usually involve two stages, the first where the lipid fraction is separated from the water-soluble proteins, which corresponds to about 40% of the total proteins and contains IgY, and the second where IgY is isolated from other soluble proteins.

The first methods described in the literature for the purification of IgY emerged in the 1950s (Jensenius et al., J. Immunol. Methods, 1981, 46, 63-68). These are based on the separation of water-soluble proteins from lipoproteins and other lipids present in the yolk by slow centrifugation processes or by successive precipitation with organic solvents, such as chloroform. However, both approaches lead to significantly low yields (Jensenius et al., J. Immunol. Methods, 1981, 46, 63-68). To date, numerous methodologies have been described, most involving precipitation processes. The first step (separation of water-soluble proteins from the lipid fraction) is usually achieved by diluting the yolk in water (De Meulenaer et al., Food Agr. Immunol., 2001, 13 (4), 275-288).

However, other techniques have been developed and tested for the removal of the lipid fraction from the fraction containing the water-soluble proteins comprising IgY. This is the case of precipitation with polyethylene glycol, natural gums, dextran sulfate, ammonium sulfate and organic solvents, methods of freezing and thawing of diluted yolk (De Meulenaer et al., Food Agr. Immunol., 2001, 13 (4), 275-288), and hydrophobic interaction and molecular exclusion chromatography (Hassl and Aspock, J. Immunol. Methods, 1988, 110:225).

The second step comprises the separation of IgY from the other soluble proteins. To this end, methods using salt/polymer precipitations (De Meulenaer et al., Food Agr. Immunol., 2001, 13 (4), 275-288) and chromatographic techniques (De Meulenaer et al., Food Agr Immunol, 2001, 13 (4), 275-288), namely affinity chromatography, ion exchange and hydrophobic interaction (De Meulenaer et al., Food Agr Immunol, 2001, 13 (4), 275-288) have been reported. Selective precipitation studies of IgY have also been performed with ammonium sulfate (De Meulenaer et al., Food Agr Immunol, 2001, 13 (4), 275-288), sodium citrate (Bizhanov et al., Scand. J. Lab. Anim. Sci., 2004, 31:121-30), lithium sulfate (Bizhanov et al., Scand. J. Lab. Anim. Sci., 2004, 31: 121-30) and ethanol (De Meulenaer et al., Food Agr Immunol, 2001, 13 (4), 275-288).

Despite the large number of works described, those using successive precipitations to separate IgY from water-soluble proteins with satisfactory purity and yield on a laboratory scale, although simple and economical, are not suitable at an industrial scale. As for chromatographic methods, these have significantly high costs that increase the commercialization price of the antibody.

Accordingly, the present disclosure intends, through an industrially applicable process, to purify chicken egg yolk IgY with a high degree of purity, high quality (with higher stability and higher activity than IgY obtained with other methods described in the literature or using commercial purification kits) and at a competitive price.

### General Description

The use of antibodies in different scientific and medicinal applications has been increasing over the last decade. However, the cost of producing high purity antibodies, namely immunoglobulin Y (IgY, an antibody present in poultry egg yolk), remains much higher than other therapies due to the lack of an effective and profitable purification method.

The present disclosure relates to a selective process for the purification of egg yolk IgY, in particular chicken egg yolk. The first step corresponds to the precipitation of the egg yolk lipid fraction, followed by a liquid-liquid extraction step by the application of aqueous biphasic systems, which allows to remove the higher molecular weight contaminating protein, and a last ultrafiltration step, allowing the removal of the lower molecular weight contaminating protein and the polymer used in the liquid-liquid extraction. The present process makes possible to obtain high quality IgY (with higher stability and higher activity than IgY obtained with other methods described in the literature or using commercial purification kits) and 95% pure in a simple, economical way, and applicable at an industrial scale.

In one of the process steps the yolk is diluted, frozen and further centrifuged, allowing the lipid fraction of the yolk (precipitate) to be removed and the water-soluble protein fraction (supernatant) to be obtained. The water-soluble protein fraction (supernatant) contains IgY of approximately 35% purity.

In another step the removal of a first contaminating protein is carried out, in particular it comprises the removal of a protein of higher molecular weight present in the aqueous fraction, in particular that corresponds to the removal of α-livetin. For this purpose, a liquid-liquid extraction method is employed by the application of aqueous biphasic systems consisting of a polymer and a salt, wherein the target protein (IgY) preferentially migrates to the polymer-rich phase and the contaminating one of higher molecular weight migrates to the opposite phase, i.e. to the salt-rich phase. The polymer-rich phase (upper phase) is recovered while the salt rich phase is discarded. The recovered phase contains IgY with a purity in the range of 12-74%. The reason why the contaminating protein migrates to the salt-rich phase is due to its lower hydrophobicity and higher affinity to more hydrophilic phases (in this case, the salt-rich phase). On the other hand, IgY, being the largest and most hydrophobic protein, migrates preferentially to the polymer-rich phase, which corresponds to the more hydrophobic phase of the aqueous biphasic system.

In yet another step, the removal of a second contaminating protein comprising removal of a lower molecular weight contaminating protein, β-livetin, and the polymer in solution by an ultrafiltration step is carried out. At the end of several centrifugation cycles, namely after 2-10 centrifugation cycles, the solution inside the 100 kDa Amicon® Ultra centrifugation filter contains 76-95% pure IgY in aqueous saline buffer solution.

The present disclosure relates to a process for purifying an antibody from egg yolk, wherein the process comprises the following steps:
obtaining an aqueous fraction from the egg yolk, wherein the aqueous fraction comprises the antibody;
extracting the antibody from said aqueous fraction by means of a biphasic aqueous solution, wherein the biphasic aqueous solution comprises a polymer and a saline buffer forming two immiscible phases, wherein the antibody has affinity to the polymer;
ultrafiltration of the immiscible phase from the previous step, wherein the immiscible phase comprises the polymer and the antibody in order to obtain the purified antibody.

In one embodiment for best results, the process for purifying an antibody from egg yolk may comprise the following steps:
obtaining an aqueous fraction from the egg yolk, wherein the aqueous fraction comprises the antibody;
extracting the antibody from said aqueous fraction by means of a biphasic aqueous solution, wherein the biphasic aqueous solution comprises a polymer and a saline buffer forming two immiscible phases, wherein the antibody has affinity to the polymer;
ultrafiltration of the immiscible phase from the previous step, wherein the immiscible phase comprises the polymer and the antibody in order to obtain the purified antibody;
wherein the step of extracting the antibody from said aqueous fraction is carried out with the biphasic aqueous solution comprising:
   5-20% (wt_{polymer}/wt_{biphasic} aqueous solution) of a polymer; preferably 5-15% (wt_{polymer}/wt_{biphasic} aqueous solution) of a polymer;
   12-30% (Wt_{saline buffer}/wt_{biphasic} aqueous solution) of a saline buffer; preferably 15-25% (Wt_{saline buffer}/wt_{biphasic} aqueous solution) of a saline buffer; and
   61-75% (wt_{aqueous fraction}/wt_{biphasic} aqueous solution) of the aqueous fraction obtained from egg yolk, preferably 61-68% (wt_{aqueous fraction}/wt_{biphasic} aqueous solution) of the aqueous fraction obtained from egg yolk.

In one embodiment for best results, the step of extracting the antibody from said aqueous fraction is carried out with the biphasic aqueous solution comprising:
5-20% (wt_{polymer/}wt_{biphasic} aqueous solution) of a polymer with a molecular weight between 1000-20000 g.mol⁻¹, preferably 5-15% (wt_{polymer}/wt_{biphasic} aqueous solution) of a polymer with a molecular weight between 1000-20000 g.mol⁻¹, even more preferably 12% (wt_{polymer/}wt_{biphasic} aqueous solution) of the polymer with a molecular weight between 1000-20000 g.mol⁻¹;
12-30% (Wt_{saline buffer}/wt_{biphasic} aqueous solution) of a saline buffer with a pH between 3.5-7.5, preferably 15-25% (Wt_{saline buffer}/wt_{biphasic} aqueous solution) of saline buffer with a pH between 3.5-7.5, even more preferably 20% (Wt_{saline buffer}/wt_{biphasic} aqueous solution) of a saline buffer with pH between 3.5-7.5;
61-75% (wt_{aqueous fraction}/wt_{biphasic} aqueous solution) of the aqueous fraction obtained from egg yolk, preferably 68% (wt_{aqueous fraction}/wt_{biphasic} aqueous solution) of the aqueous fraction obtained from egg yolk.

In one embodiment for best results, the polymer may have a molecular weight between 4000-15000 g.mol⁻¹, preferably between 5000-12000 g.mol⁻¹, more preferably between 8000-10000 g.mol⁻¹.

In one embodiment for best results, the polymer may be selected from the following list: polyethylene glycol, polypropylene glycol, dextran or polyvinylpyrrolidone, preferably polyethylene glycol.

In one embodiment and for even better results, the polymer is polyethylene glycol with a molecular weight of 10000 g.mol⁻¹.

In one embodiment, the saline buffer used may have a pH between 4.5-6.5 and may be selected from the following list: C₆H₈O₇/ Na₂HPO₄ buffer, C₆H₈O₇/K₂HPO₄ buffer or C₆H₈O₇/ Na₃C₆H₅O₇ buffer, preferably C₆H₈O₇/K₂HPO₄ buffer with a pH at 4.5.

In one embodiment, the step of obtaining an aqueous fraction from egg yolk may comprise diluting the egg yolk 5-20 times with water, preferably 5-10 times with water, even more preferably 7 times in water.

In one embodiment, the step of obtaining an aqueous fraction from egg yolk may be carried out at a pH between 4-6, preferably a pH of 5.

In one embodiment, the diluted yolk may be frozen at a temperature between -10 and 0 °C, preferably between -10 and -6 °C, even more preferably between -8 and -6 °C.

In one embodiment, the diluted yolk may be frozen for 12-20 hours, preferably between 12-16 hours.

In one embodiment, the frozen diluted yolk may be thawed at room temperature for 1-3 hours, preferably between 1-2 hours.

In one embodiment, the thawed diluted yolk may be centrifuged in order to collect the aqueous fraction from the diluted egg yolk, preferably wherein the thawed diluted yolk is centrifuged at 400-10000 g, preferably 740-6650 g, even more preferably 2960-4650 g.

In one embodiment, the step of extracting the antibody may be carried out at 10-30 °C for 1-12 hours, preferably 25 °C for 4 hours.

In one embodiment, the step of ultrafiltration of the antibody may be carried out 2-10 times, preferably 3-8 times, even more preferably 4-6 times, with saline buffer at pH 5-8, preferably at a pH 7, selected from the following list: Na₂HPO₄/KH₂PO₄ buffer, K₂HPO₄/KH₂PO₄ buffer, Na₂HPO₄/NaH₂PO₄ buffer, C₆H₈O₇/Na₂HPO₄ buffer or C₆H₈O₇/K₂HPO₄ buffer, preferably Na₂HPO₄/NaH₂PO₄ buffer.

In one embodiment for best results, the step of ultrafiltration of the antibody may be carried out with a filter with pores between 10-150 kDa, preferably 50-120 kDa, even more preferably 100 kDa.

In one embodiment, the antibody is IgY.

In one embodiment, the egg yolk may be chicken egg yolk.

This disclosure also relates to an antibody, in particular IgY, obtainable by the process described in any one of the preceding claims.

### Description of the Figures

For an easier understanding of the present disclosure, figures are herein attached, which represent preferred embodiments which however are not intended to limit the object of the present disclosure.
**Figure 1****:** Schematic representation of the process for the purification of IgY from chicken egg yolk.
**Figure 2****:** Size-exclusion chromatogram of the water-soluble protein fraction obtained after the first purification step and containing IgY 35% pure.
**Figure 3****:** Impact of pH value on aqueous biphasic systems consisting of polymer + saline buffer + H₂O, between 20-30 °C.
**Figure 4****:** Impact of the molecular weight of the polymer on aqueous biphasic systems consisting of polymer + saline buffer + H₂O, between 20-30 °C.
**Figure 5**: Size-exclusion chromatogram after liquid-liquid extraction and containing approximately IgY 74% pure.
**Figure 6****:** Schematic representation of an ultrafiltration filter used to remove the polymer and lower molecular weight contaminating protein in solution with IgY.
**Figure 7****:** Schematic representation of a system used to recover purified IgY after the last ultrafiltration step.
**Figure 8****:** Size-exclusion chromatogram of the IgY solution 95% pure obtained after the complete purification process.
**Figure 9****:** Comparison of IgY concentration obtained by various methods described in the literature, with commercial kits and with the proposed process. IgY (Chicken) ELISA Kit, Abnova.

### Detailed Description

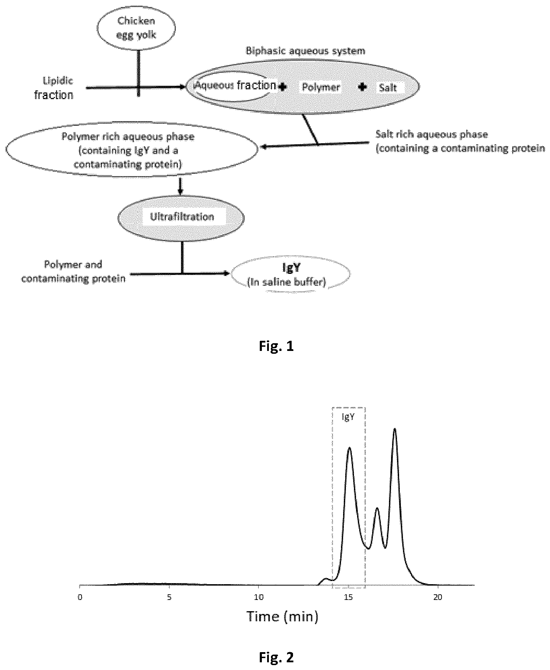

The present disclosure relates to a process comprising several steps for the purification of IgY from chicken egg yolk, in particular as summarized in **Figure 1****.**

In one embodiment, the removal of the lipid fraction from the chicken egg yolk is carried out. The egg yolk should be diluted about 5-20 times with distilled water, preferably it can be diluted between 5-10 times with distilled water, even more preferably it can be diluted 7 times with distilled water; and the obtained solution should be stirred until a homogeneous mixture is achieved. Then hydrochloric acid should be added at a concentration of 0.5-3 M, preferably 0.5-2 M until a pH value between 4-6 is obtained. The diluted yolk should then be frozen at a temperature between -10-0 °C, preferably between -10 and -6 °C for a period of time between 12-20 hours. To thaw the diluted yolk, it should be placed in water at room temperature for a period of time between 1-3 hours. In order to perform the removal of the lipid fraction from the diluted yolk, the solution is centrifuged at a rotation speed between 400-10000 g, preferably 740-6650 g, preferably 2960-4650 g, for a period of time between 1-15 hours, preferably 1-7 hours, even more preferably 1-2 hours, at a temperature between 2-10 °C, preferably between 4-10 °C. At the end of the centrifugation a yellow precipitate (lipid fraction) and a transparent supernatant corresponding to the water-soluble protein fraction are obtained, the latter fraction containing the IgY. The supernatant is recovered and filtered through a 0.2-1.2 µm diameter pore filter to remove any residues that have not been properly disposed of in centrifugation while the yellow precipitate is discarded. The aqueous solution after filtration contains 35% pure IgY and two contaminating proteins, namely α-livetin and β-livetin, as shown in the size-exclusion chromatogram of **Figure 2****.**

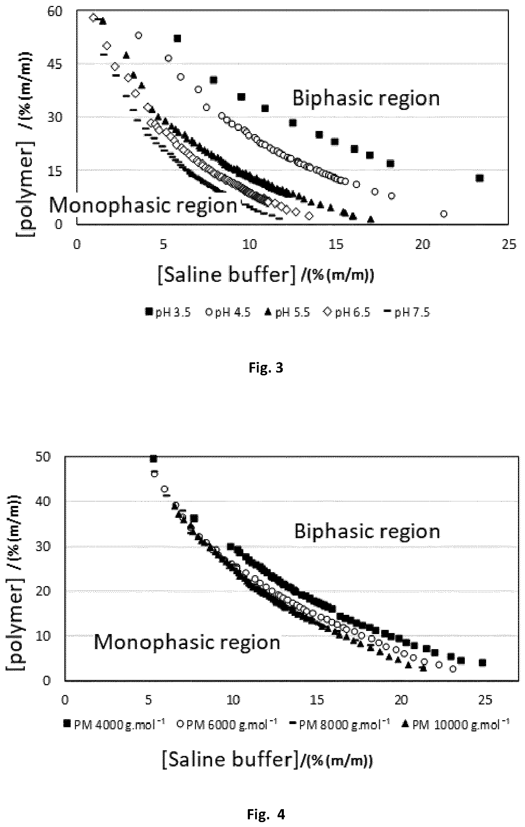

In one embodiment, after removal of the lipid fraction of the chicken egg yolk, the removal of the larger molecular weight contaminating protein from the aqueous solution is carried out. This step comprises increasing the purity of IgY which is present in the aqueous solution obtained with the first step. For this, a liquid-liquid extraction is used by the application of an aqueous biphasic system consisting of a polymer and a salt in which the target protein (IgY) preferably migrates to the polymer-rich phase. The reason why the target protein preferentially migrates to the polymer-rich phase is due to the following reason: IgY, being the largest and most hydrophobic protein, preferentially migrates to the polymer-rich phase, which corresponds to the most hydrophobic phase of the aqueous biphasic system. Liquid-liquid extraction is accomplished by the addition of appropriate quantities of components able to form two phases **(****Figures 3** and **4****),** wherein the appropriate amounts/concentrations are determined by knowing the phase diagrams that have been added, as well as the purity, yield, *EE*% and K results for IgY for the various systems and conditions studied.

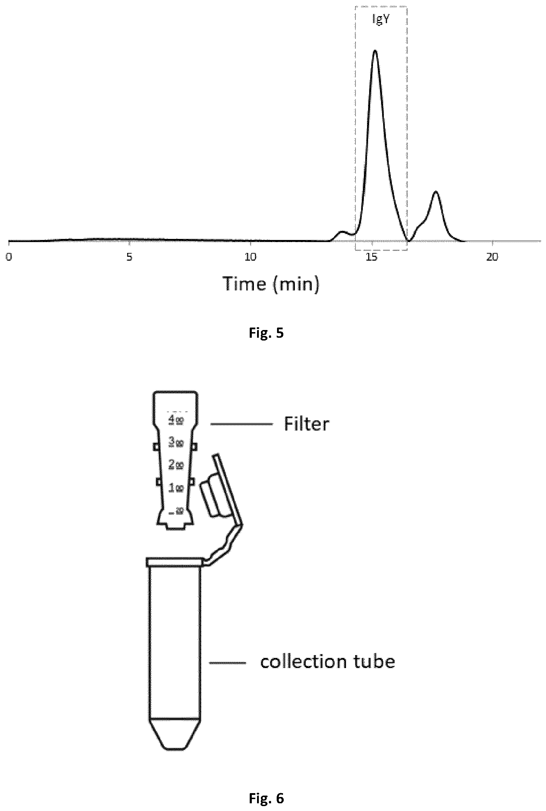

As an example, liquid-liquid extraction may be carried out using mixtures with 5-20% (w/w), preferably 10-20% (w/w) of a polymer with a molecular weight from 1000 to 20000 g.mol⁻¹, 12-20% (w/w) preferably 15-20% (w/w) of saline buffer, preferably C₆H₈O₇/K₂HPO₄ with a pH value of 3.5-7.5, preferably 4.5-6.5 and 60-75% (w/w) corresponding to the aqueous solution containing the IgY and the two contaminating proteins. Mixing of all components should be carried out sequentially in no defined order and should be gently stirred until a homogeneous solution is obtained. The solution should be allowed to stand at a temperature between 10-30 °C, preferably 20-30 °C for a time period between 1-12 hours, preferably between 2-9 hours, even more preferably between 3-6 hours, so that the two liquid phases are formed and the balance between them is established. After rest time the two phases will be formed and the upper phase corresponds to the polymer-rich phase and the lower phase refers to the salt-rich phase. The separation of the phases must be carried out extremely carefully and thoroughly so that there is no contamination of both phases. The polymer-rich phase (upper phase) is recovered while the salt rich phase is discarded. The recovered phase contains IgY, with a purity ranging between 12-74% **(Tables 1 to 4),** and the lowest molecular weight contaminating protein, β-livetin, as can be observed, the best result in the size-exclusion chromatogram in **Figure 5****.** Thus, at this stage, the higher molecular weight contaminating protein α-livetin is completely removed and the purity of IgY is increased from 35 to 74%.

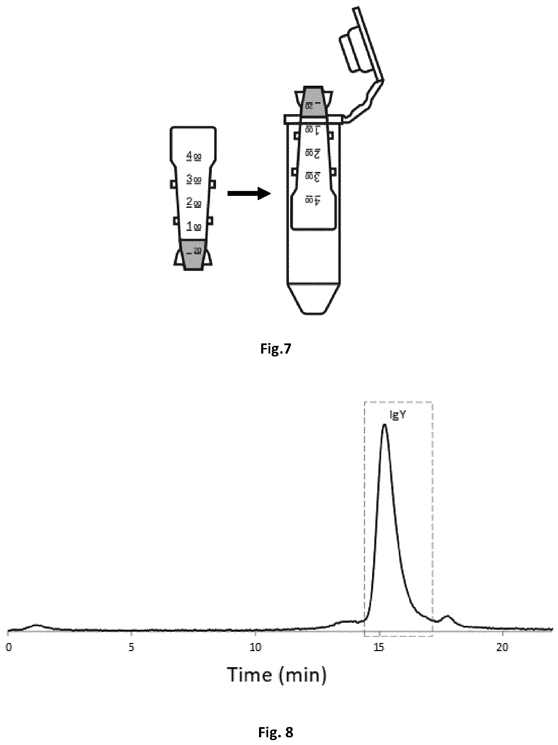

In one embodiment, the lower molecular weight contaminating protein and the polyethylene glycol used in forming the aqueous biphasic system are removed. In the last step the last contaminating protein in solution with IgY is removed, namely the lower molecular weight contaminating protein, β-livetin, is removed and also the polymer which is in solution is removed by an ultrafiltration step as shown in the diagram in **Figure 6****.** For this, the polymer-rich upper aqueous phase (containing IgY and the contaminating protein) is ultrafiltrated 2-10 times, preferably 3-8 times, even more preferably 4-7 times, with filters with a pore size from 10 to 100 kDa, preferably 50-100 kDa, at a rotation speed of 1000-15000 g, preferably 10000-14000 g for 10-20 minutes. Between each centrifugation cycle about 250 to 500 µL of saline buffer with a pH value between 5-8, preferably 5 to 7, is added to the inside of the filter and the solution that is excluded to the outside of the filter is discarded, namely sodium phosphate buffer is added. At the end of the number of cycles mentioned above, the solution inside the filter is recovered by centrifuging it at a rotation speed of 500-2000 g, preferably 500-1000 g for 2-10 minutes as shown in the scheme of **Figure 7****.** This solution contains the purified IgY, with 77-95% purity **(Table 5)** in aqueous saline buffer solution with a pH value between 5 and 7, preferably 7, as shown, with the best result in size-exclusion chromatogram of **Figure 8****.**

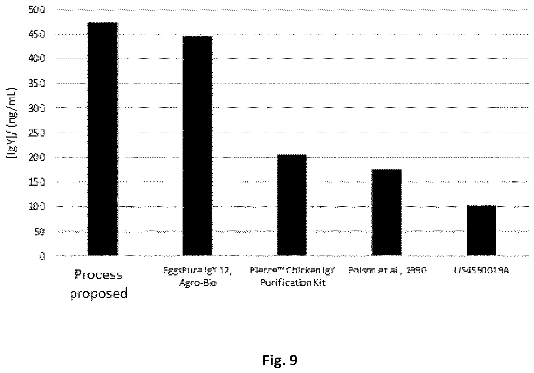

In one embodiment, immunoassays (ELISA) were finally applied to IgY obtained by the process now disclosed, and compared to IgY activity obtained with other methods described in the literature and using commercial purification kits - **Figure 9****.** The results obtained show that the proposed purification process allows to obtain higher stability and activity of IgY versus the IgY obtained with other methods described in the literature or using commercial purification kits. Methods in the literature and commercial purification kits allow to obtain IgY of similar purity but with lower IgY activity.

The present disclosure allows IgY to be used in two distinct areas. IgY, if purified from hyperimmune eggs, can be used as a biopharmaceutical to treat a wide range of diseases, and can be used in diagnostic kits replacing the regularly used IgG. On the other hand, IgY purified from non-hyperimmune eggs may be used in the formulation of food supplements as it may strengthen the immune system of humans and other animals or be used for research and diagnosis.

The product obtained (IgY) in the present disclosure has a high purity, in particular higher than 95%, and is obtained at a very competitive cost, thus allowing its larger scale use.

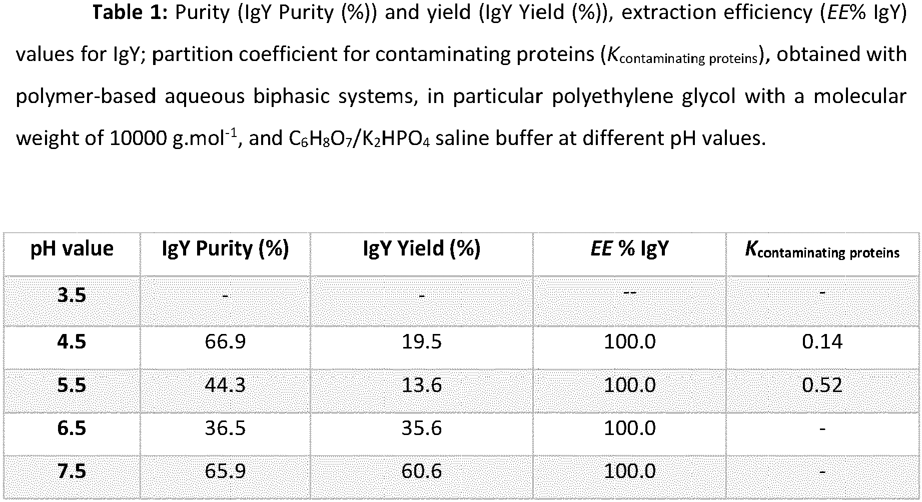

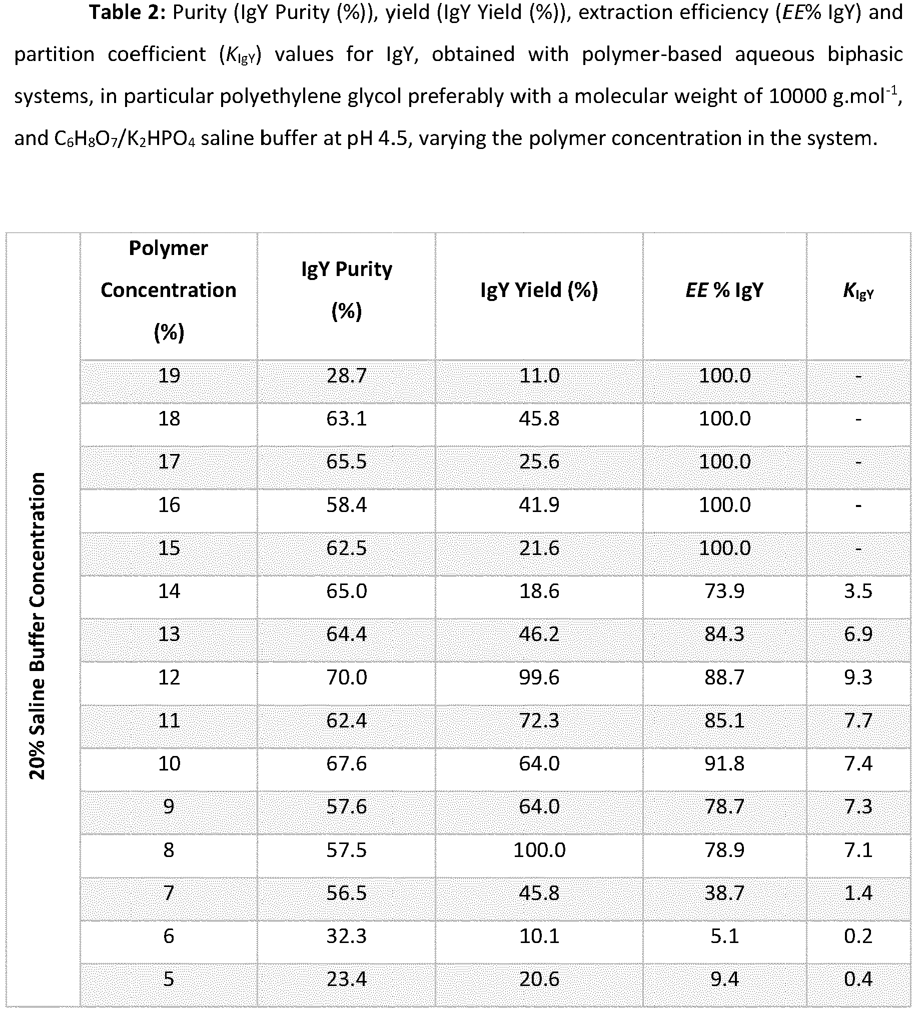

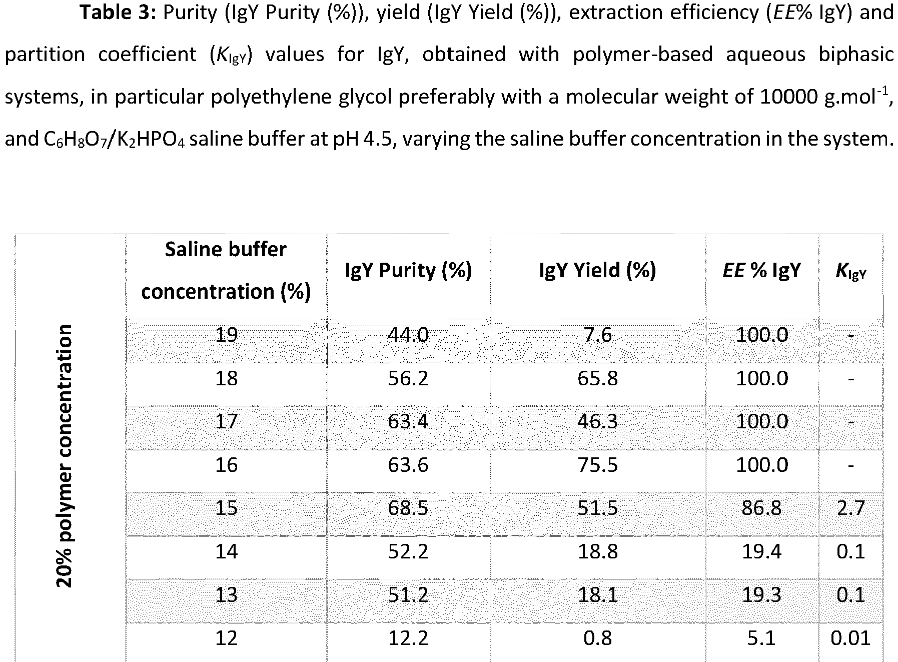

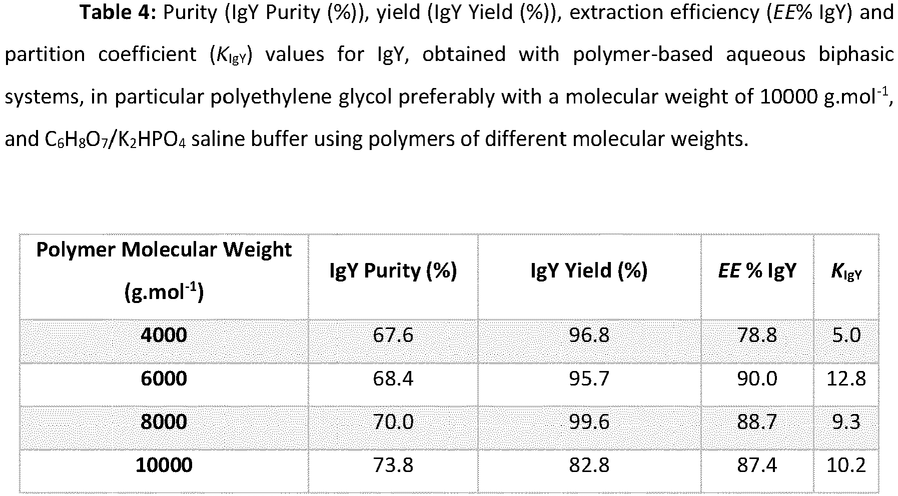

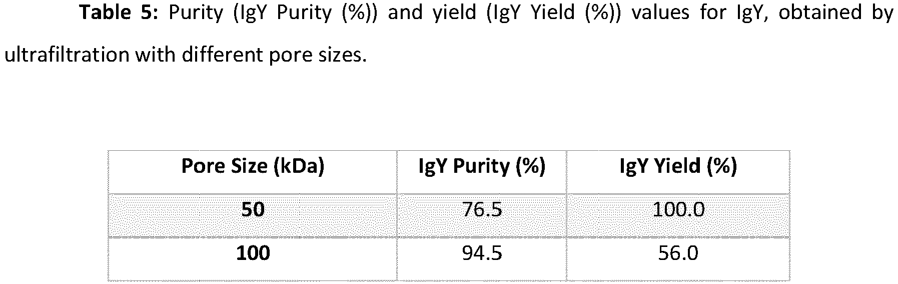

Although in the present disclosure only particular embodiments of the invention have been represented and described, the person skilled in the art will know how to introduce modifications and replace technical features for equivalent ones, depending on the requirements of each situation, without departing from the scope of protection defined by the appended claims. The embodiments presented are combinable with each other.

The following claims further define embodiments.

## Claims

1. Process for purifying an antibody from egg yolk, wherein the process comprises the following steps:
obtaining an aqueous fraction from the egg yolk, wherein the aqueous fraction comprises the antibody;
extracting the antibody from said aqueous fraction by means of a biphasic aqueous solution, wherein the biphasic aqueous solution comprises a polymer and a saline buffer forming two immiscible phases, wherein the antibody has affinity to the polymer;
ultrafiltration of the immiscible phase from the previous step, wherein the immiscible phase comprises the polymer and the antibody in order to obtain the purified antibody;
wherein the step of extracting the antibody from said aqueous fraction is carried out with the biphasic aqueous solution comprising:
5-20% (wt_{polymer}/wt_{biphasic} aqueous solution) of a polymer;
12-30% (wt_{saline buffer}/wt_{biphasic} aqueous solution) of a saline buffer; and
61-75% (wt_{aqueous fraction}/wt_{biphasic} aqueous solution) of the aqueous fraction obtained from egg yolk.

2. Process according to the preceding claim, wherein the step of extracting the antibody from the said aqueous fraction is carried out with the biphasic aqueous solution comprising:
5-15% (wt_{polymer}/wt_{biphasic} aqueous solution) of a polymer;
15-25% (wt_{saline buffer/}wt_{biphasic} aqueous solution) of a saline buffer; and
61-68% (wt_{aqueous fraction}/wt_{biphasic} aqueous solution) of the aqueous fraction obtained from egg yolk.

3. Process according to any one of the preceding claims, wherein the step of extracting the antibody from the said aqueous fraction is carried out with the biphasic aqueous solution comprising:
5-20% (wt_{polymer}/wt_{biphasic} aqueous solution) of a polymer with a molecular weight between 1000-20000 g.mol⁻¹;
12-30% (wt_{saline buffer}/wt_{biphasic} aqueous solution) of a saline buffer with a pH between 3.5-7.5; and
61-75% (wt_{aqueous fraction}/wt_{biphasic} aqueous solution) of the aqueous fraction obtained from egg yolk.

4. Process according to any one of the preceding claims, wherein the step of extracting the antibody from the said fraction is carried out with the biphasic aqueous solution comprising:
5-15% (wt_{polymer/}wt_{biphasic} aqueous solution) of the polymer with a molecular weight between 1000-20000 g.mol⁻¹;
15-25% (wt_{saline buffer}/wt_{biphasic aqueous solution}) of the saline buffer with a pH between 3.5-7.5; and
68% (wt_{aqueous fraction}/wt_{biphasic aqueous solution}) of the aqueous fraction obtained from egg yolk.

5. Process according to any one of the preceding claims, wherein the step of extracting the antibody from the said fraction is carried out with the biphasic aqueous solution comprising:
12% (wt_{polymer/}wt_{biphasic} aqueous solution) of the polymer with a molecular weight between 1000-20000 g.mol⁻¹;
20% (wt_{saline buffer}/wt_{biphasic aqueous solution}) of the saline buffer with a pH between 3.5-7.5; and 68% (wt_{aqueousfraction}/wt_{biphasic aqueous solution}) of the aqueous fraction obtained from egg yolk.

6. Process according to any one of the preceding claims, wherein the polymer has a molecular weight between 4000-15000 g.mol⁻¹, preferably between 5000-12000 g.mol⁻¹, more preferably between 8000-10000 g.mol⁻¹.

7. Process according to any one of the preceding claims, wherein the polymer is selected from the following list: polyethylene glycol, polypropylene glycol, dextran or polyvinylpyrrolidone, preferably polyethylene glycol.

8. Process according to any one of the preceding claims, wherein the polymer is polyethylene glycol having a molecular weight of 10000 g.mol⁻¹.

9. Process according to any one of the preceding claims, wherein the saline buffer has a pH between 4.5-6.5.

10. Process according to any one of the preceding claims, wherein the saline buffer is selected from the following list: C₆H₈O₇/ Na₂HPO₄ buffer, C₆H₈O₇/K₂HPO₄ buffer or C₆H₈O₇/ Na₃C₆H₅O₇ buffer, preferably C₆H₈O₇/K₂HPO₄ buffer with a pH of 4.5.

11. Process according to any one of the preceding claims, wherein the step of obtaining an aqueous fraction from egg yolk comprises diluting the egg yolk 5-20 times with water, preferably 5-10 times with water, even more preferably 7 times in water.

12. Process according to any one of the preceding claims, wherein the step of obtaining an aqueous fraction from the egg yolk is carried out with a pH between 4-6, preferably with a pH of 5.

13. Process according to any one of the preceding claims 11-12, wherein the diluted yolk is frozen at a temperature between -10 and 0 °C, preferably between -10 and -6 °C, even more preferably between -8 and -6 °C.

14. Process according to the preceding claim, wherein the diluted yolk is frozen for 12-20 hours, preferably between 12-16 hours.

15. Process according to any one of the preceding claims, wherein the frozen diluted yolk is thawed at room temperature for 1-3 hours, preferably between 1-2 hours.

16. Process according to the preceding claim, wherein the thawed diluted yolk is centrifuged so as to collect the aqueous fraction from the diluted egg yolk, preferably wherein the thawed diluted yolk is centrifuged at 400-10000 g, preferably 740-6650 g, even more preferably 2960-4650 g, preferably for a period of time between 2-15 hours, preferably 1-7 hours, even more preferably 1-2 hours, and at a temperature between 2-10 °C, preferably 4-10 °C.

17. Process according to any one of the preceding claims, wherein the step of extracting the antibody is carried out at 10-30 °C for 1-12 hours, preferably at 25 °C for 4 hours.

18. Process according to any one of the preceding claims, wherein the step of ultrafiltering the antibody is carried out 2-10 times, preferably 3-8 times, even more preferably 4-6 times, with saline buffer at pH 5-8, preferably at a pH 7, selected from the following list: Na₂HPO₄/KH₂PO₄ buffer, K₂HPO₄/KH₂PO₄ buffer, Na₂HPO₄/NaH₂PO₄ buffer, C₆H₈O₇/Na₂HPO₄ buffer or C₆H₈O₇/K₂HPO₄ buffer, preferably Na₂HPO₄/NaH₂PO₄ buffer.

19. Process according to any one of the preceding claims, wherein the ultrafiltration step of the antibody is carried out with a filter with pores between 10-150 kDa, preferably 50-120 kDa, even more preferably 100 kDa.

20. Process according to any one of the preceding claims, wherein the antibody is IgY.

21. Process according to any one of the claims, wherein the egg yolk is chicken egg yolk.

22. Antibody obtainable by the process described in any one of the preceding claims.
